# FASCICULE DE BREVET EUROPEEN

(11) **EP 1 353 663 B1**
(45) Date de publication et mention de la délivrance du brevet: **02.09.2009**
(21) Numéro de dépôt: 02700354.0
(22) Date de dépôt: 22.01.2002
(51) Int. Cl.: A61K 31/216, A61K 9/20, A61K 9/28

(54) **COMPRIMES DE FENOFIBRATE**
FENOFIBRATTABLETTEN
FENOFIBRATE TABLETS

(30) Priorité: 22.01.2001 FR 0100833
(43) Date de publication de la demande: 22.10.2003
(73) Titulaire: LABORATOIRES FOURNIER SA, 21000 Dijon (FR)
(72) Inventeur: BLOUQUIN, Pascale, F-21240 Talant (FR); REGINAULT, Philippe, F-21121 Fontaine les Dijon (FR)
(74) Mandataire: Hubert, Philippe
(86) Numéro de dépôt international: PCT/FR2002/000245
(87) Numéro de publication internationale: WO 2002/056881

(56) Documents cités:
- EP-A- 0 793 958
- WO-A-98/31361

## Description

La présente invention concerne une nouvelle formulation galénique du fénofibrate administrable par voie orale, son procédé d'obtention ainsi que les médicaments fabriqués à partir de ces formulations.

Le fénofibrate (DCI) de la famille des fibrates est un principe actif de médicament connu depuis de nombreuses années en raison de son efficacité pour diminuer les taux de triglycérides et de cholestérol dans le sang. De ce fait, le fénofibrate est largement prescrit dans de nombreux pays quand il est nécessaire de diminuer le risque athérogène.

On sait également que, pour obtenir un effet hypocholestérolémiant satisfaisant, il est souhaitable de maintenir un taux circulant d'acide fénofibrique (qui est le métabolite actif du fénofibrate) de l'ordre de 6 à 10 mg/l. Un tel taux est notamment obtenu avec une dose unitaire de fénofibrate de 300 mg en gélule (cf. Drugs 40 (2) p 260-290 (1990)).

On sait aussi qu'il existe des variations importantes des taux circulants en fonction des pathologies observées chez les patients. D'une façon générale pour tous les médicaments, il est préférable de maintenir un taux circulant du métabolite actif nécessaire pour obtenir l'effet thérapeutique recherché tout en faisant absorber au patient une quantité minimale de principe actif. C'est pourquoi, on recherche des formulations présentant la plus haute biodisponibilité possible afin d'optimiser la posologie et de limiter d'éventuels effets secondaires du principe actif.

Compte tenu de ces éléments, on comprend que la formulation galénique d'un principe actif absorbé par voie orale présente une grande importance pour obtenir l'effet thérapeutique dans des conditions optimales.

Le fénofibrate a été commercialisé à l'origine sous forme de gélules dosées à 100 mg de principe actif, avec une posologie de 3 gélules par jour, puis sous forme de gélules dosées à 300 mg de principe actif, prescrites à raison d'une gélule par jour. Les études mentionnées précédemment font état, après administration d'une gélule dosée à 300 mg à des volontaires sains, de taux circulant d'acide fénofibrique maximum d'environ 6 à 9 mg/l et d'une aire sous courbe de 145 à 170 mg/l.h.

Une autre formulation divulguée dans le document EP 330 532, et commercialisée en France sous la marque LIPANTHYL^{®} 200M, résulte d'un procédé consistant à comicroniser le fénofibrate avec un composé tensioactif solide de façon à obtenir un mélange intime et finement divisé des deux produits. Ce type de formulation permet de réduire la posologie à 200 mg/jour en une seule prise pour obtenir des concentrations plasmatiques d'acide fénofibrique très proches de celles obtenues avec une prise de 300 mg de fénofibrate non co-micronisé (Journal International de Médecine (1991) n° 206 p 48-50). Cette formulation présente une biodisponibilité améliorée de l'ordre de 30 %, par rapport à la formulation d'origine.

Un autre type de formulation a été proposé dans le document FR 2 494 112. Il s'agit ici de microgranules dans lesquelles un noyau neutre, constitué de saccharose et d'amidon, est enrobé de fénofibrate micronisé puis recouvert d'une couche de protection microporeuse. La posologie préconisée, dans ce cas, est de 250 mg/jour, ce qui correspond à une biodisponibilité intermédiaire par rapport aux formulations précédentes.

On connaît également, par le document EP 757 911 un procédé de préparation d'une formulation de fénofibrate consistant à réaliser une solution du principe actif dans l'éther monoéthylique du diéthylèneglycol et à conditionner cette solution dans des capsules molles. Selon les résultats mentionnés dans ce document, l'administration de 100 mg/jour de fénofibrate permettrait d'obtenir les concentrations plasmatiques d'acide fénofibrique requises pour assurer l'efficacité du médicament. En d'autres termes, cette formulation présenterait une biodisponibilité double de celle de la formulation décrite dans le document EP 330 532. Une telle formulation soulève toutefois le problème résultant de l'administration en quantité relativement importante du solvant par voie orale. En effet, selon les formulations décrites, la prise de 100 mg de fénofibrate correspond à la prise simultanée de 1500 mg d'un éther du diéthylèneglycol. Or, le fénofibrate étant un hypolipidémiant destiné à être prescrit sur des périodes prolongées, l'utilisation d'une telle formulation reviendrait à faire absorber quotidiennement et régulièrement au patient 1,5 g d'un éther du diéthylèneglycol dont les effets biologiques ne sont pas totalement neutres (cf. Food Cosmet. Toxicol. (1968) 6 (6) p689-705 ; Arzneim. Forsch (1978) 28(9) p 1571-1579 ; Occup. Hyg. (1996), 2 (1-6, Proceedings of the Int. Symposium on Health Hazards of glycol Ethers, 1994), 131-151).

Le document WO 00/57918 propose également une formulation de fénofibrate en solution sous la forme d'un préconcentré destiné à former une émulsion en présence d'une phase aqueuse, et par conséquent dans l'estomac du patient après administration. Ce type de formulation nécessite cependant l'utilisation de capsules étanches et résistantes à la matière huileuse.

Le document WO 00/30615 propose aussi une formulation liquide de fénofibrate constituée de particules micronisées de fénofibrate maintenues en suspension dans un liquide en présence de tensioactifs.

On connaît également, selon 5ème Congrès Intern. Technol. Pharm. Vol. 3 (1989) p 190-199, une formulation se présentant sous la forme d'une solution de fénofibrate dans le diméthylisosorbide en mélange avec un agent dispersant. Selon les essais mentionnés, on obtient essentiellement une biodisponibilité qui est beaucoup moins dépendante de la présence d'aliments dans le milieu gastrique, ce qui constitue déjà un progrès par rapport à la forme classique en gélules pour lesquelles la biodisponibilité serait variable de 26 % à jeun à 89 % après repas.

Une formulation proche de la précédente est proposée dans le document EP 904 781, qui préconise de préparer un co-fondu de fénofibrate et d'un dispersant solide tel que la croscarmellose ou la polyvinylpyrolidone.

Selon le document CA 2253 769, le fénofibrate est formulé sous forme d'un co-précipité avec un excipient soluble dans l'eau, tel que notamment l'hydroxypropylméthylcellulose. Le procédé de fabrication d'un tel co-précipité nécessite toutefois l'utilisation de solvants organiques qui doivent être parfaitement éliminés dans le produit fini et qui posent également des problèmes de sécurité pendant le séchage par atomisation. Une formulation proche de celle-ci est décrite dans le document EP 761208.

On connaît également, par le document CA 2270 306 des gélules ou des comprimés contenant du fénofibrate co-micronisé avec du lactose, qui présentent une biodisponibilité améliorée du principe actif par comparaison avec une formulation dans laquelle le fénofibrate serait micronisé seul, puis mélangé avec du lactose.

On a également décrit d'autres formes sèches en gélules de fénofibrate comme par exemple dans le document EP 793 958 qui propose de mélanger le fénofibrate avec de la PVP (polyvinylpyrrolidone), de la PVP réticulée et éventuellement d'autres excipients, de granuler le mélange ainsi obtenu avec une solution d'un agent tensioactif, puis de sécher le granulé que l'on met en gélules ; ou encore dans le document FR 2783 421 qui, selon un procédé très proche du précédent, préconise de microniser le fénofibrate, de le granuler en présence d'un milieu liquide comprenant un agent tensioactif, de l'eau et un alcool hydromiscible puis de sécher le granulat ainsi obtenu, lequel peut être ensuite mélangé à d'autres excipients puis mis en gélules.

Par ailleurs, on connaît par le document FR 2 758 459 une composition sous forme de granulés ou de comprimés dans lesquels le fénofibrate, sous forme micronisée, est associé à un polymère hydrophile (notamment la polyvinylpyrrolidone) et éventuellement à un tensioactif. Les résultats obtenus avec un granulé conforme à cette formulation contenant un tensioactif, montrent une dissolution plus rapide du fénofibrate dans des tests de laboratoire. Une étude pharmacocinétique comparative montre également une biodisponibilité améliorée, notamment en considérant les paramètres de la concentration plasmatique en acide fénofibrique et de l'aire sous courbe.

Cette dernière formulation peut être présentée sous forme de comprimés, mais dans ce cas, les comprimés en résultant présentent un poids unitaire d'environ 750 mg pour un dosage de 160 mg de fénofibrate. De tels comprimés, en raison de leur dimension relativement importante, sont difficiles à administrer, ce qui en limite l'intérêt.

D'une façon générale, on sait que la forme comprimée est plus avantageuse que la forme en gélules dans la mesure où :
- d'une part, à poids égal, un comprimé est de dimension plus réduite qu'une gélule ; et
- d'autre part, les cadences de production industrielle des comprimés sont plus élevées que celles des formes en gélules, ou encore en capsules molles.

De plus, la forme comprimé évite l'utilisation de matières premières d'origine animale telle que la gélatine qui est le constituant essentiel des gélules.

Dans ces conditions, la présente invention a pour but de résoudre le problème technique consistant en la fourniture d'une nouvelle formulation du fénofibrate présentant une bonne biodisponibilité et qui permette de réaliser des comprimés de dimension réduite, plus faciles à administrer que ceux décrits dans l'état de la technique et notamment dans le document FR 2 758 459.

On a plus particulièrement cherché une formulation contenant une quantité réduite d'excipients, tout en conservant une aptitude à former des comprimés réalisables à l'échelle industrielle et présentant de bonnes caractéristiques de résistance à l'écrasement et de friabilité, ainsi qu'une bonne biodisponibilité.

Il a ainsi été découvert qu'il était possible d'atteindre cet objectif et, contrairement à l'enseignement de l'art antérieur, d'obtenir des comprimés renfermant plus de 50 % en poids de principe actif et présentant une biodisponibilité équivalente à celle des gélules commerciales contenant un comicronisat de fénofibrate et d'un tensioactif solide.

Ainsi, selon un premier aspect, l'invention a pour objet une composition pharmaceutique sous forme d'un comprimé administrable par voie orale susceptible d'être obtenu par compression d'un mélange comprenant :
a) un granulat contenant :
   - du fénofibrate micronisé ;
   - 1 à 5% en poids, rapporté au poids du fénofibrate, d'un agent tensioactif ;
   - au moins un excipient solide choisi parmi l'amidon, la cellulose ou leurs dérivés, à l'exception des disaccharides en C₁₂ ;
   ledit granulat étant obtenu par granulation du mélange à l'aide d'une solution aqueuse de polyvinylpyrrolidone
b) de la polyvinylpyrrolidone réticulée
c) éventuellement des agents d'écoulement ou de lubrification,
la quantité de fénofibrate étant supérieure à 50 % en poids, exprimée par rapport au poids du comprimé.

Une telle formulation permet de résoudre le problème technique précité de façon particulièrement avantageuse puisqu'elle conduit à des comprimés réalisables industriellement, présentant d'excellentes caractéristiques de résistance à l'écrasement et de friabilité et ayant une taille suffisamment petite pour être acceptée par les patients, ce qui est particulièrement important dans le cadre d'un traitement hypocholestérolémique de longue durée.

Par ailleurs, les comprimés obtenus montrent d'une façon inattendue une biodisponibilité équivalente à celle des gélules contenant le principe actif sous forme comicronisée avec l'agent tensio-actif.

Selon une forme préférée de réalisation de l'invention, l'agent tensioactif précité est solide et se présente sous forme d'un comicronisat avec le fénofibrate.

Selon une autre forme de réalisation préférée de l'invention, l'agent tensioactif précité est introduit dans le granulat dans la solution de granulation avec la polyvinylpyrrolidone.

Selon une caractéristique particulière de l'invention, le comprimé contient entre 50 et 250 mg de fénofibrate.

Selon une autre caractéristique particulière de l'invention, l'agent tensio-actif solide précité est le laurylsulfate de sodium.

Selon une autre caractéristique particulière, l'un des excipients solides précités est l'amidon prégélatinisé, qui est présent en une quantité en poids de 15 à 40 % du poids du fénofibrate.

Selon encore une autre caractéristique particulière, l'un des excipients est la cellulose microcristalline, qui est présent en une quantité en poids de 5 à 30 % du poids du fénofibrate.

Selon une autre forme de réalisation actuellement préférée de l'invention, le comprimé précité présente un enrobage constitué par une pellicule d'un vernis de protection contre l'humidité, de préférence à base de polymère hydrodispersible.

Selon un deuxième aspect, l'invention a pour objet un procédé de préparation d'une composition pharmaceutique de fénofibrate sous forme d'un comprimé administrable par voie orale tel que décrit précédemment.

Selon un premier mode de réalisation, ce procédé est essentiellement caractérisé en ce qu'il comprend :
- la comicronisation d'une quantité efficace de fénofibrate et d'un agent tensio-actif solide, lequel est utilisé en une quantité comprise entre 1 et 5 % en poids rapportée au poids du fénofibrate ;
- le mélange sous forme de poudres, du produit ainsi obtenu avec au moins un excipient solide choisi parmi l'amidon, la cellulose ou leurs dérivés, à l'exception des disaccharides en C₁₂ ;
- la granulation du mélange de poudres ainsi obtenu à l'aide d'une solution aqueuse de polyvinylpyrrolidone ;
- le mélange du granulat ainsi obtenu avec de la polyvinylpyrrolidone réticulée et éventuellement des agents d'écoulement ou de lubrification ; étant précisé que la quantité de fénofibrate est supérieure à 50 % en poids, exprimée par rapport au poids du comprimé ;
- la compression du mélange ainsi réalisé ; et
- éventuellement, le pelliculage du comprimé ainsi obtenu par un vernis protecteur, de préférence à base de polymère hydrodispersible.

Selon un second mode de réalisation, ce procédé est essentiellement caractérisé en ce qu'il comprend :
- le mélange, sous forme de poudres, de fénofibrate micronisé avec au moins un excipient solide choisi parmi l'amidon, la cellulose ou leurs dérivés, à l'exception des disaccharides en C₁₂ ;
- la granulation du mélange de poudres ainsi obtenu à l'aide d'une solution aqueuse de polyvinylpyrrolidone et d'un agent tensioactif, ledit agent tensioactif étant en une quantité comprise entre 1 et 5 % en poids par rapport au poids du fénofibrate;
- le mélange du granulat ainsi obtenu avec de la polyvinylpyrrolidone réticulée et éventuellement des agents d'écoulement ou de lubrification ; étant précisé que la quantité de fénofibrate est supérieure à 50 % en poids, exprimée par rapport au poids du comprimé ;
- la compression du mélange ainsi réalisé ; et
- éventuellement, le pelliculage du comprimé ainsi obtenu par un vernis protecteur, de préférence à base de polymère hydrodispersible.

### Description détaillée

Selon l'invention, on propose donc une nouvelle forme galénique du fénofibrate, administrable par voie orale, sous forme d'un comprimé, telle que définie précédemment, laquelle est obtenue par compression d'une formulation nouvelle.

Cette formulation constituée du granulat précité en mélange avec de la polyvinylpyrrolidone réticulée, présente une bonne aptitude à la compression et permet d'obtenir de façon particulièrement originale des comprimés de taille réduite contenant plus de 50 % en poids de principe actif. Ainsi, il est possible selon l'invention de réaliser un comprimé présentant un poids unitaire inférieur à 400 mg pour une posologie de 200 mg de fénofibrate en une seule administration.

Selon l'invention, le fénofibrate est tout d'abord micronisé seul ou comicronisé avec un agent tensioactif solide de façon à obtenir une poudre dont la granulométrie moyenne est de préférence inférieure à 30 µm et de préférence encore inférieure à 10 µm. Plus précisément, dans le cas d'une comicronisation, le fénofibrate cristallisé et l'agent tensioactif solide en poudre sont mélangés puis le mélange est broyé dans un microniseur, par exemple selon la technologie décrite dans le document EP 330 532 en permettant ainsi d'obtenir un mélange intime des deux constituants et d'améliorer sensiblement la biodisponibilité du fénofibrate. La micronisation du fénofibrate ou du mélange fénofibrate-agent tensioactif est effectuée avantageusement dans un microniseur à jet d'air, qui permet d'obtenir une poudre dont la taille moyenne des particules est de l'ordre de 5 à 10 µm sans échauffement des produits.

Dans le cas de la comicronisation du mélange fénofibrate/agent tensioactif, la quantité de tensioactif solide est de l'ordre de 1 à 5 % de la quantité de fénofibrate, et préférentiellement de l'ordre de 3 à 4 %. De façon pratique, on choisit un agent tensioactif solide, ionique ou non ionique. Parmi les agents tensioactifs susceptibles d'être utilisés, on préfère le laurylsulfate de sodium Le fénofibrate et l'agent tensioactif comicronisés sont ensuite mélangés avec au moins un excipient pulvérulent, et le mélange est granulé de préférence à l'aide d'une solution de polyvinylpyrrolidone dans l'eau.

Dans le cas de l'utilisation de fénofibrate micronisé seul, celui-ci est mélangé avec au moins un excipient pulvérulent et l'agent tensioactif (également utilisé en une quantité de l'ordre de 1 à 5 % en poids, exprimée par rapport au poids du fénofibrate) est introduit préférentiellement en solution avec la polyvinylpyrrolidone dans l'eau du liquide de granulation. Ce mode de préparation est appliqué notamment pour un agent tensioactif liquide ou visqueux, comme par exemple les polysorbates ou les esters d'acides gras à chaîne moyenne.

Parmi les excipients pulvérulents susceptibles d'être utilisés dans le cadre de l'invention, on préfère l'amidon et/ou la cellulose ou leurs dérivés, comme, par exemple, la carboxyméthylcellulose, à l'exception des disaccharides en C₁₂. L'amidon prégélatinisé qui est un bon agent de désintégration et la cellulose microcristalline qui permet d'obtenir une bonne cohésion et de réduire les risques de clivage du comprimé sont particulièrement préférés.

A l'inverse de l'enseignement de l'art antérieur le plus proche, le comprimé selon l'invention ne contient pas de disaccharides en C₁₂, en particulier de lactose qui est souvent présent dans les formulations connues de comprimés. Il a été découvert, de façon inattendue que, dans le cas d'un comprimé à base de fénofibrate, le remplacement du lactose par des excipients pulvérulents convenablement choisis permettait d'améliorer la biodisponibilité du principe actif tout en diminuant sensiblement la quantité des excipients nécessaires et d'obtenir ainsi, de façon tout à fait avantageuse un comprimé plus petit.

D'une façon préférée, ces excipients sont ajoutés en une quantité totale correspondant à environ 30 à 70 %, et préférentiellement 45 à 55 %, du poids de fénofibrate, chacun des excipients, considéré indépendamment, étant présent en une quantité comprise entre 5 et 40 %, préférentiellement entre 10 et 35 % en poids rapporté au poids de fénofibrate.

Le mélange pulvérulent constitué par le fénofibrate et les excipients précités est granulé par exemple dans un mélangeur à pales ou tout autre appareil permettant d'obtenir un granulé, au moyen d'une solution liante comprenant avantageusement de la polyvinylpyrrolidone dans l'eau. Pour cette granulation, on utilise de préférence une polyvinylpyrrolidone de poids moléculaire moyen compris entre 25000 et 100000. Le granulat obtenu est ensuite calibré par passage au travers d'une grille dont l'ouverture de maille est avantageusement de 1 à 2,5 mm, puis séché, de préférence dans un séchoir granulateur à lit fluidisé.

Le granulé sec ainsi obtenu peut être ensuite mélangé à une phase externe solide constituée d'agents de formulation tels que des agents lubrifiants, des agents d'écoulement, des liants ou des agents de désintégration. Parmi ces agents, on préfère la polyvinylpyrrolidone réticulée qui accélère la désintégration du comprimé en milieu aqueux et/ou le stéarate de magnésium qui permet la lubrification du mélange.

Les excipients constituant la phase externe du granulat sont généralement présents en une quantité correspondant à environ 5 à 25 % et préférentiellement de 8 à 15 % du poids de fénofibrate.

Selon un mode de réalisation préféré de l'invention, la phase externe comprend de la polyvinylpyrrolidone réticulée en tant qu'agent de désintégration, avantageusement en une quantité en poids de 6 à 18 % et préférentiellement 10 à 14 %, rapportée au poids de fénofibrate.

D'une façon générale, la polyvinylpyrrolidone réticulée, encore dénommée crospovidone, présente un réseau polymérique branché avec des masses moléculaires supérieures à 1 000 000.

Le mélange du granulat et de ces excipients est avantageusement homogénéisé par exemple dans un mélangeur à pales à axe horizontal ou vertical. Le mélange ainsi obtenu est ensuite transformé en comprimés calibrés pour contenir unitairement 50 à 250 mg de fénofibrate, chaque comprimé pesant ainsi entre environ 100 et 500 mg. L'opération de compression est conduite à l'aide d'une machine alternative ou, préférentiellement, rotative équipée de poinçons dont le diamètre est de l'ordre de 5 à 12 mm, préférentiellement 8 à 10 mm, de façon à obtenir des comprimés de petite taille.

Les comprimés ainsi obtenus peuvent être utilisés directement en thérapeutique, mais, compte tenu du caractère hydrophile de certains excipients, il est préférable, dans le but d'assurer une meilleure conservation du médicament, d'enrober les comprimés à l'aide d'un vernis protecteur de préférence à base de polymères hydrodispersibles. Néanmoins ce pelliculage n'est pas indispensable, la bonne conservation du comprimé pouvant être assurée par d'autres moyens comme par exemple par un conditionnement limitant les échanges de vapeur d'eau avec l'extérieur. Le pelliculage peut être réalisé de façon classique, selon des procédés connus de l'homme de l'art, par exemple par pulvérisation d'une solution de polymère filmogène sur les comprimés placés dans une turbine. Ce pelliculage permet également, le cas échéant, de colorer les comprimés par ajout d'un pigment coloré à la solution de polymère de pelliculage.

L'exemple de préparation suivant, ainsi que les résultats d'études cliniques obtenus permettront de mieux apprécier l'objet de l'invention.

### Exemple 1

a) Préparation du comicronisat de fénofibrate :
   On mélange 6,8 kg de fénofibrate cristallisé et 238 g de laurylsulfate de sodium dans un mélangeur à palettes à axe horizontal pendant 6 mn, puis ce mélange homogène est broyé finement dans un microniseur à jet d'air de façon à obtenir un co-micronisat dont la taille moyenne des particules est de l'ordre de 5 à 10 µm.
b) Préparation du granulat de phase interne
   On charge 7,038 kg du comicronisat obtenu selon la préparation ci-dessus, 2,312 kg d'amidon prégélatinisé (référence LYCATAB^{®} PGS commercialisé par la société ROQUETTE) et 1,190 kg de cellulose microcristalline (référence AVICEL^{®} PH 102 de la société FMC Corp.) dans un mélangeur à palettes à axe horizontal (LÖDIGE) et on mélange ces poudres pendant 3 minutes. Le mélange est ensuite granulé dans le mélangeur par pulvérisation d'une solution de polyvinylpyrrolidone obtenu à partir de 357 g de PVP (référence Kollidon K30 commercialisé par la société BASF) et 3,625 l d'eau purifiée. Le granulé ainsi obtenu est calibré par passage sur un granulateur oscillant (ERWEKA) équipé d'une grille dont l'ouverture de maille est 2,5 mm. Le granulé ainsi tamisé est ensuite transféré dans un sécheur à lit fluidisé et séché par passage d'air à 50-60 °C.
c) préparation du mélange à comprimer
   On mélange 10,897 kg du granulé obtenu selon le procédé ci-dessus, 833 g de polyvinylpyrrolidone réticulée (référence POLYPLASDONE^{®} XL 10 commercialisé par la société GAF) et 170 g de stéarate de magnésium dans un mélangeur à palettes à axe horizontal (LÖDIGE), pendant 6 à 10 minutes. On obtient ainsi le granulé mélangé à la phase externe, prêt pour la compression.
d) préparation des comprimés
   Le mélange obtenu ci-dessus est comprimé dans une machine rotative équipée sur 6 postes de poinçons 10R10. Le réglage est effectué de façon à obtenir des comprimés dont la masse unitaire est de 350 mg, ce qui correspond à un dosage de 200 mg de fénofibrate par comprimé. Les comprimés obtenus présentent une dureté d'environ 89N.
e) pelliculage
   Les comprimés obtenus selon l'opération ci-dessus sont placés dans une turbine en acier inoxydable (ERWEKA) équipée d'un pistolet de pulvérisation, d'une ventilation soufflante à air chaud et d'une aspiration. La rotation de la turbine est réglée sur 16 tours/mn et on pulvérise une solution de 75 g de polymère filmogène (référence OPADRY OYS^{®} commercialisé par la société COLORCON) dans 1,425 kg d'éthanol à 80 %. La pulvérisation dure environ 2 heures. Les comprimés pelliculés obtenus sont ensuite refroidis et conditionnés. Chaque comprimé présente un diamètre de 10 mm et un poids d'environ 360 mg, pour un dosage de 200 mg de fénofibrate.

### Exemple 2

a) Préparation du granulat de phase interne
   On mélange pendant environ 6 minutes, dans un mélangeur à axe horizontal (LÖDIGE), :
   - 3,4 kg de fénofibrate micronisé (diamètre moyen des particules : 7 µm)
   - 1,156 kg d'amidon prégélatinisé (LYCATAB^{®} PGS)
   - 0,595 kg de cellulose microcristalline (AVICEL^{®} PH 102).

   Ce mélange est ensuite granulé dans le mélangeur par pulvérisation d'une solution de 178,5 g de polyvinylpyrrolidone (KOLLIDON K 30) et 119 g de polysorbate 80 (TWEEN^{®} 80 obtenu auprès de la société Uniqema) dans 1,813 l d'eau purifiée. La pulvérisation dure environ 30 mn. Le granulé obtenu est calibré par passage sur un granulateur oscillant (ERWEKA) équipé d'une grille de 2,5 mm d'ouverture de maille. Le granulé tamisé est ensuite séché dans un sécheur à lit fluidisé (GLATT) avec une température d'introduction d'air de 50-60°C.
b) Préparation du mélange à comprimer
   On mélange 1,859 kg du granulat obtenu selon le procédé ci-dessus, 142 g de polyvinylpyrrolidone réticulée (référence POLYPLASDONE^{®} XL 10 commercialisé par la société GAF) et 29 g de stéarate de magnésium dans un mélangeur à palettes à axe horizontal (LÔDIGE), pendant 6 minutes. On obtient ainsi le granulé mélangé à la phase externe, prêt pour la compression.
c) préparation des comprimés
   Le mélange obtenu ci-dessus est comprimé dans une machine alternative équipée d'un poinçon 10R10. Le réglage est effectué de façon à obtenir des comprimés dont la masse unitaire est de 350 mg, ce qui correspond à un dosage de 200 mg de fénofibrate par comprimé. Les comprimés obtenus présentent une dureté d'environ 89N.
d) pelliculage
   Les comprimés obtenus selon l'opération ci-dessus sont placés dans une turbine en acier inoxydable équipée d'un pistolet de pulvérisation, d'une ventilation soufflante à air chaud et d'une aspiration. La rotation de la turbine est réglée sur 16 tours/mn et on pulvérise une solution de 12,8 g de polymère filmogène (référence OPADRY OYS^{®}) dans 243 g d'éthanol à 80 %. La pulvérisation dure environ 1 heure. Les comprimés pelliculés obtenus sont ensuite refroidis et conditionnés. Chaque comprimé présente un diamètre de 10 mm et un poids d'environ 360 mg, pour un dosage de 200 mg de fénofibrate.

### Exemple comparatif

A titre de comparaison, on a aussi préparé un lot de comprimés de fénofibrate en suivant un protocole classique pour l'homme du métier, dans lequel les excipients constituant la phase interne de la préparation 1b de l'exemple 1 ci-dessus ont été remplacés par 2,550 kg de lactose, 952 g de carboxyméthylamidon sodique et 595 g de cellulose microcristalline, et en utilisant, en tant qu'excipients de la phase externe de la préparation 1c 238 g de PVP XL10 et 170 g de stéarate de magnésium ; la compression et le pelliculage ayant été réalisés de façon analogue à la fabrication de l'exemple 1, avec un dosage de 200 mg de fénofibrate pour un comprimé de 360 mg pelliculé. Les comprimés obtenus présentent une dureté d'environ 72 à 75 N.

### Etude pharmacocinétique

Les comprimés selon l'exemple 1 de l'invention, ainsi que les comprimés obtenus selon l'exemple comparatif ont été évalués d'un point de vue pharmacocinétique au cours d'études cliniques sur homme sain.

La première étude a été conduite sur 6 sujets afin de comparer les gélules LIPANTHYL^{®} 200M (formulation commercialisée en France) et les comprimés obtenus selon l'exemple comparatif. Le traitement a été administré, par voie orale, 30 mn après le petit déjeuner et l'efficacité du traitement a été évaluée par dosage du taux d'acide fénofibrique dans le sérum en fonction du temps. Ces mesures ont permis de calculer l'aire sous courbe (AUC 0-∞), la concentration maximale atteinte (Cmax), le temps correspondant à cette concentration maximale (Tmax) ainsi que la demi-vie du produit (T ½). Les résultats obtenus sont notés dans le tableau suivant :

**Tableau I**

| | AUC _{0 -infini} (µg /ml.h) | C max (µg /ml) | Tmax (heure) | T ½ (heure) |
|---|---|---|---|---|
| Comprimé comparatif | 102.7 | 5.36 | 4.2 | 20.3 |
| LIPANTHYL 200M | 137.2 | 8.50 | 4.7 | 18.7 |
| IC 90% log 1/2 | 0.63 -0.88 | 0.53 - 0.74 | - | - |
| Conclusion | Non bioéquivalent | Non bioéquivalent | - | - |

| | | | | |
|---|---|---|---|---|
| IC est l'intervalle de confiance. | | | | |

Cette première étude montre que le comprimé selon l'exemple comparatif, bien que comprenant la même quantité de fénofibrate n'est pas bioéquivalent à la gélule LIPANTHYL^{®} 200M et présente une biodisponibilité inférieure.

La seconde étude, conduite dans des conditions analogues à l'étude précédente sur 9 sujets, a permis de comparer le comprimé de l'exemple 1 selon l'invention et la gélule de LIPANTHYL^{®} 200 M. Les résultats sont reportés dans le tableau II suivant :

**Tableau II**

| | AUC ₀₋ₜ (µg /ml.h) | C max (µg /ml) | Tmax (heure) | T ½ ( heure) |
|---|---|---|---|---|
| LIPANTHYL^{®} 200M | 113.8 | 6.97 | 3.9 | 19.90 |
| Comprimé Exemple 1 | 115.1 | 6.86 | 4.5 | 18.4 |
| IC 90% 2/1 | 0.95 -1.08 | ANOVA NS | | |
| Conclusion | 1 et 2 bioéquivalents | | - | - |

| | | | | |
|---|---|---|---|---|
| t : temps du dernier point quantifiable | | | | |

Ces résultats montrent des valeurs très proches pour les 2 formes galéniques, qui sont significatives d'une bioéquivalence de ces deux formules contenant chacune 200 mg de fénofibrate.

Les comprimés obtenus selon les exemples 1 et 2 selon l'invention ont été comparés sur le plan de leur vitesse de dissolution. Pour ce test on a utilisé les comprimés non pelliculés et on a mesuré la quantité de fénofibrate dissous en fonction du temps, dans une solution 0,02M de laurylsulfate de sodium, à une température de 37°C, placée dans l'appareil dissolutest et sous une agitation réglée à 100 tours/mn ; selon ce protocole, les comprimés obtenus selon les exemples 1 et 2 présentent les profils de dissolution reportés dans le tableau III (les résultats sont exprimés en pourcentage de fénofibrate dissous, calculé par rapport à la quantité totale contenue dans le comprimé).

**TABLEAU III**

| Temps (mn) | Exemple 1 | Exemple 2 |
|---|---|---|
| 0 | 0 | 0 |
| 10 | 37,1 | 44,8 |
| 20 | 63,1 | 69,1 |
| 30 | 74,1 | 77,5 |
| 40 | 79,6 | 82,6 |
| 50 | 83,9 | 85,6 |
| 60 | 86,2 | 88,1 |

Les valeurs obtenues montrent des profils de dissolution tout à fait semblables, significatifs d'une équivalence des deux comprimés.

Les comprimés selon l'invention permettent donc d'obtenir une efficacité de traitement équivalente à celle des gélules LIPANTHYL^{®} 200M avec les avantages de supprimer la présence de la gélatine de la gélule, de proposer une unité de prise plus compacte et plus facile à avaler et enfin de permettre une cadence de production supérieure à celle des gélules.

Ces comprimés sont utilisables de façon analogue aux gélules de LIPANTHYL^{®} 200 M pour traiter les hypercholestérolémies et les hypertriglycéridémies.

## Revendications

1. Comprimé de fénofibrate **caractérisé en ce qu'**il est obtenu par compression d'un mélange comprenant :
a) un granulat contenant :
- du fénofibrate micronisé ;
- 1 à 5% en poids, rapporté au poids du fénofibrate, d'un agent tensioactif ;
- au moins un excipient solide choisi parmi l'amidon, la cellulose ou leurs dérivés, à l'exception des disaccharides en C₁₂ ;
ledit granulat étant obtenu par granulation du mélange à l'aide d'une solution aqueuse depolyvinylpyrrolidone
b) de la polyvinylpyrrolidone réticulée
c) éventuellement des agents d'écoulement ou de lubrification, la quantité de fénofibrate étant supérieure à 50 % en poids, exprimée par rapport au du poids du comprimé.

2. Comprimé de fénofibrate selon la revendication 1, **caractérisé en ce que** l'agent tensioactif précité utilisé pour l'obtention du granulat est solide et se présente sous forme d'un comicronisat avec le fénofibrate.

3. Comprimé de fénofibrate selon la revendication 1 **caractérisé en ce que** l'agent tensioactif précité utilisé pour l'obtention du granulat est présent dans la solution de granulation contenant la polyvinylpyrrolidone.

4. Comprimé de fénofibrate selon l'une des revendications 1 à 3, **caractérisé en ce qu'**il contient entre 50 et 250 mg de fénofibrate.

5. Comprimé de fénofibrate selon l'une des revendications 1 à 4, **caractérisé en ce que** l'agent tensioactif est le laurylsulfate de sodium.

6. Comprimé de fénofibrate selon l'une des revendications 1 à 5, **caractérisé en ce que** l'un des excipients solides précité est l'amidon prégélatinisé, qui est présent en une quantité en poids de 15 à 40 % du poids du fénofibrate.

7. Comprimé de fénofibrate selon l'une des revendications 1 à 5, **caractérisé en ce que** l'un des excipients est la cellulose microcristalline, qui est présent en une quantité en poids de 5 à 30 % du poids du fénofibrate.

8. Comprimé de fénofibrate selon l'une des revendications 1 à 7, **caractérisé en ce que** la polyvinylpyrrolidone présente un poids moléculaire moyen de l'ordre de 25000 à 100000.

9. Comprimé de fénofibrate selon l'une des revendications 1 à 8, **caractérisé en ce que** la polyvinylpyrrolidone réticulée est présente en une quantité en poids de 6 à 18 % et de préférence de 10 à 14 % du poids du fénofibrate.

10. Comprimé de fénofibrate selon l'une des revendications 1 à 9, **caractérisé en ce qu'**il présente un pelliculage d'un vernis protecteur de préférence à base de polymère hydrodispersible.

11. Procédé de préparation d'une composition pharmaceutique de fénofibrate sous forme d'un comprimé administrable par voie orale, **caractérisé en ce qu'**il comprend :
- la comicronisation d'une quantité efficace de fénofibrate et d'un agent tensio-actif solide, lequel est utilisé en une quantité comprise entre 1 et 5 % en poids rapportée au poids du fénofibrate ;
- le mélange sous forme de poudres, du produit ainsi obtenu avec au moins un excipient solide choisi parmi l'amidon, la cellulose ou leurs dérivés, à l'exception des disaccharides en C₁₂ ;
- la granulation du mélange de poudres ainsi obtenu à l'aide d'une solution aqueuse de polyvinylpyrrolidone ;
- le mélange du granulat ainsi obtenu avec de la polyvinylpyrrolidone réticulée et éventuellement des agents d'écoulement ou de lubrification ; étant précisé que la quantité de fénofibrate est supérieure à 50 % en poids, exprimée par rapport au poids du comprimé ;
- la compression du mélange ainsi réalisé ; et
- éventuellement le pelliculage du comprimé ainsi obtenu par un vernis protecteur, de préférence à base de polymère hydrodispersible.

12. Procédé de préparation d'une composition pharmaceutique de fénofibrate sous forme d'un comprimé administrable par voie orale, essentiellement **caractérisé en ce qu'**il comprend :
- le mélange, sous forme de poudres, de fénofibrate micronisé avec au moins un excipient solide choisi parmi l'amidon, la cellulose ou leurs dérivés, à l'exception des disaccharides en C₁₂ ;
- la granulation du mélange de poudres ainsi obtenu à l'aide d'une solution aqueuse de polyvinylpyrrolidone et d'un agent tensioactif, ledit agent tensioactif étant en une quantité comprise entre 1 et 5 % en poids par rapport au poids du fénofibrate;
- le mélange du granulat ainsi obtenu avec de la polyvinylpyrrolidone réticulée et éventuellement des agents d'écoulement ou de lubrification ; étant précisé que la quantité de fénofibrate est supérieure à 50 % en poids, exprimée par rapport au poids du comprimé ;
- la compression du mélange ainsi réalisé ; et
- éventuellement, le pelliculage du comprimé ainsi obtenu par un vernis protecteur, de préférence à base de polymère hydrodispersible.

13. Procédé selon l'une des revendications 11 ou 12, **caractérisé en ce que** la polyvinylpyrrolidone réticulée est utilisée en une quantité comprise entre 6 et 18 %, de préférence entre 10 et 14 % en poids, rapportée au poids du fénofibrate.

14. Procédé selon l'une des revendications 11 à 13, **caractérisé en ce que** le(les) excipient(s) solide(s) précité(s) est(sont) utilisé(s) en une quantité comprise entre 5 et 40 %, de préférence entre 10 et 35 % en poids, rapportée au poids du fénofibrate.

## Claims

1. Fenofibrate tablet, **characterized in that** it is obtained by compressing a mixture comprising:
a) granules containing:
- micronized fenofibrate
- 1 to 5% by weight, based on the weight of fenofibrate, of a surfactant;
- at least one solid excipient selected from starch, cellulose and derivatives thereof, with the exception of C₁₂ disaccharides, said granules being obtained by granulating the mixture with the aid of an aqueous solution of polyvinylpyrrolidone;
b) crosslinked polyvinylpyrrolidone; and
c) optionally flow aids or lubricants,
the amount of fenofibrate being greater than 50% by weight, expressed relative to the weight of the tablet.

2. Fenofibrate tablet according to claim 1, **characterized in that** the aforementioned surfactant used to obtain the granules is solid and takes the form of a comicronizate with the fenofibrate.

3. Fenofibrate tablet according to claim 1, **characterized in that** the aforementioned surfactant used to obtain the granules is present in the granulating solution containing the polyvinylpyrrolidone.

4. Fenofibrate tablet according to one of claims 1 to 3, **characterized in that** it contains between 50 and 250 mg of fenofibrate.

5. Fenofibrate tablet according to one of claims 1 to 4, **characterized in that** the surfactant is sodium laurylsulfate.

6. Fenofibrate tablet according to one of claims 1 to 5, **characterized in that** one of the aforementioned solid excipients is pregelatinized starch, which is present in an amount of 15 to 40% of the weight of the fenofibrate.

7. Fenofibrate tablet according to one of claims 1 to 5, **characterized in that** one of the excipients is microcrystalline cellulose, which is present in an amount of 5 to 30% of the weight of the fenofibrate.

8. Fenofibrate tablet according to one of claims 1 to 7, **characterized in that** the polyvinylpyrrolidone has an average molecular weight in the order of 25,000 to 100,000.

9. Fenofibrate tablet according to one of claims 1 to 8, **characterized in that** the crosslinked polyvinylpyrrolidone is present in an amount of 6 to 18% and preferably of 10 to 14% of the weight of the fenofibrate.

10. Fenofibrate tablet according to one of claims 1 to 9, **characterized in that** it has a coating consisting of a film of protective varnish, preferably based on a water-dispersible polymer.

11. Process for the preparation of a pharmaceutical fenofibrate composition in the form of a tablet for oral administration, **characterized in that** it comprises:
- the comicronization of an effective amount of fenofibrate and a solid surfactant, which is used in an amount of between 1 and 5% by weight, based on the weight of fenofibrate;
- the mixing, in the form of powders, of the resulting product with at least one solid excipient selected from starch, cellulose and derivatives thereof, with the exception of C₁₂ disaccharides;
- the granulation of the resulting mixture of powders with the aid of an aqueous solution of polyvinylpyrrolidone;
- the mixing of the resulting granules with crosslinked polyvinylpyrrolidone and optionally flow aids or lubricants,
it being specified that the amount of fenofibrate is greater than 50% by weight, expressed relative to the weight of the tablet;
- the compression of the mixture produced; and
- optionally the film-coating of the resulting tablet with a protective varnish, preferably based on a water-dispersible polymer.

12. Process for the preparation of a pharmaceutical fenofibrate composition in the form of a tablet for oral administration, essentially **characterized in that** it comprises:
- the mixing, in the form of powders, of micronized fenofibrate with at least one solid excipient selected from starch, cellulose and derivatives thereof, with the exception of C₁₂ disaccharides;
- the granulation of the resulting mixture of powders with the aid of an aqueous solution of polyvinylpyrrolidone and a surfactant, said surfactant being in an amount of between 1 and 5% by weight, based on the weight of fenofibrate;
- the mixing of the resulting granules with crosslinked polyvinylpyrrolidone and optionally flow aids or lubricants,
it being specified that the amount of fenofibrate is greater than 50% by weight, expressed relative to the weight of the tablet;
- the compression of the mixture produced; and
- optionally the film-coating of the resulting tablet with a protective varnish, preferably based on a water-dispersible polymer.

13. Process according to claim 11 or 12, **characterized in that** the crosslinked polyvinylpyrrolidone is used in an amount of between 6 and 18% and preferably of between 10 and 14% by weight, based on the weight of fenofibrate.

14. Process according to one of claims 11 to 13, **characterized in that** the aforementioned solid excipient(s) is (are) used in an amount of between 5 and 40% and preferably of between 10 and 35% by weight, based on the weight of fenofibrate.

## Patentansprüche

1. Fenofibrat-Tablette, **dadurch gekennzeichnet, daß** sie durch Verpressen eines Gemischs erhalten wird, das folgendes umfaßt:
a) ein Granulat enthaltend:
- mikronisiertes Fenofibrat,
- 1 bis 5 Gew.-% - bezogen auf das Gewicht des Fenofibrats - eines oberflächenaktiven Mittels,
- wenigstens ein festes Bindemittel, welches aus Stärke, Cellulose oder deren Derivaten, mit Ausnahme der C₁₂-Disaccharide, ausgewählt ist,
wobei das Granulat durch Granulieren des Gemischs mit Hilfe einer wäßrigen Polyvinylpyrrolidon-Lösung erhalten wird,
b) vernetztes Polyvinylpyrrolidon,
c) eventuell Fließ- oder Schmiermittel,
wobei die Menge an Fenofibrat mehr als 50 Gew.-%, bezogen auf das Gewicht der Tablette beträgt.

2. Fenofibrat-Tablette nach Anspruch 1, **dadurch gekennzeichnet, daß** das vorgenannte oberflächenaktive Mittel, das für den Erhalt des Granulats verwendet wird, fest ist und in Form eines Co-Mikronisats mit dem Fenofibrat vorliegt.

3. Fenofibrat-Tablette nach Anspruch 1, **dadurch gekennzeichnet, daß** das vorgenannte oberflächenaktive Mittel, das für den Erhalt des Granulats verwendet wird, in der das Polyvinylpyrrolidon enthaltenden Granulierungslösung vorhanden ist.

4. Fenofibrat-Tablette nach einem der Ansprüche 1 bis 3, **dadurch gekennzeichnet, daß** sie 50 bis 250 mg Fenofibrat enthält.

5. Fenofibrat-Tablette nach einem der Ansprüche 1 bis 4, **dadurch gekennzeichnet, daß** das oberflächenaktive Mittel Natriumlaurylsulfat ist.

6. Fenofibrat-Tablette nach einem der Ansprüche 1 bis 5, **dadurch gekennzeichnet, daß** eines der festen vorgenannten Bindemittel vorgelatinierte Stärke ist, die in einer Gewichtsmenge zwischen 15 und 40 % des Gewichts des Fenofibrats vorhanden ist.

7. Fenofibrat-Tablette nach einem der Ansprüche 1 bis 5, **dadurch gekennzeichnet, daß** eines der Bindemittel mikrokristalline Cellulose ist, die in einer Gewichtsmenge zwischen 5 und 30 % des Gewichts des Fenofibrats vorliegt.

8. Fenofibrat-Tablette nach einem der Ansprüche 1 bis 7, **dadurch gekennzeichnet, daß** das Polyvinylpyrrolidon ein durchschnittliches Molekulargewicht in der Größenordnung von 25 000 bis 100 000 aufweist.

9. Fenofibrat-Tablette nach einem der Ansprüche 1 bis 8, **dadurch gekennzeichnet, daß** das vernetzte Polyvinylpyrrolidon in einer Gewichtsmenge zwischen 6 und 18 % und vorzugsweise zwischen 10 und 14 % des Gewichts des Fenofibrats vorliegt.

10. Fenofibrat-Tablette nach einem der Ansprüche 1 bis 9, **dadurch gekennzeichnet, daß** sie einen Überzug aus einem Schutzlack, vorzugsweise auf der Basis eines wasserdispergierbaren Polymers aufweist.

11. Verfahren zur Herstellung einer pharmazeutischen Fenofibrat-Zusammensetzung in Form einer auf oralem Weg verabreichbaren Tablette, **dadurch gekennzeichnet, daß** es folgendes umfaßt:
- das gemeinsame Mikronisieren einer wirkungsvollen Menge an Fenofibrat und eines festen oberflächenaktiven Mittels, das in einer Menge zwischen 1 und 5 Gew.-%, bezogen auf das Gewicht des Fenofibrats verwendet wird,
- das Mischen des so erhaltenen Produkts, in Form von Pulvern, mit wenigstens einem festen Bindemittel, das aus Stärke, Cellulose oder deren Derivaten, mit Ausnahme der C₁₂-Disaccharide, ausgewählt ist,
- das Granulieren des so erhaltenen Pulvergemischs mit Hilfe einer wäßrigen Polyvinylpyrrolidon-Lösung,
- das Mischen des so erhaltenen Granulats mit vernetztem Polyvinylpyrrolidon und eventuell mit Fließ- oder Schmiermitteln,
wobei die Menge an Fenofibrat mehr als 50 Gew.-%, bezogen auf das Gewicht der Tablette beträgt,
- das Verpressen des so erzeugten Gemischs, und
- eventuell das Überziehen der so erhaltenen Tablette mit einem Schutzlack, vorzugsweise auf der Basis eines wasserdispergierbaren Polymers.

12. Verfahren zur Herstellung einer pharmazeutischen Fenofibrat-Zusammensetzung in Form einer auf oralem Weg verabreichbaren Tablette, das im wesentlichen **dadurch gekennzeichnet ist, daß** es folgendes umfaßt:
- das Mischen des mikronisierten Fenofibrats, in Form von Pulvern, mit wenigstens einem festen Bindemittel, das aus Stärke, Cellulose oder deren Derivaten, mit Ausnahme der C₁₂-Disaccharide, ausgewählt ist,
- das Granulieren des so erhaltenen Pulvergemischs mit Hilfe einer wäßrigen Polyvinylpyrrolidon-Lösung und einem oberflächenaktiven Mittel, wobei das oberflächenaktive Mittel in einer Menge zwischen 1 und 5 Gew.-%, bezogen auf das Gewicht des Fenofibrats vorliegt,
- das Mischen des so erhaltenen Granulats mit vernetztem Polyvinylpyrrolidon und eventuell mit Fließ- oder Schmiermitteln,
wobei die Menge an Fenofibrat mehr als 50 Gew.-%, bezogen auf das Gewicht der Tablette beträgt,
- das Verpressen des so erzeugten Gemischs, und
- eventuell das Überziehen der so erhaltenen Tablette mit einem Schutzlack, vorzugsweise auf der Basis eines wasserdispergierbaren Polymers.

13. Verfahren nach einem der Ansprüche 11 oder 12, **dadurch gekennzeichnet, daß** das vernetzte Polyvinylpyrrolidon in einer Menge zwischen 6 und 18, vorzugsweise zwischen 10 und 14 Gew.-%, bezogen auf das Gewicht des Fenofibrats verwendet wird.

14. Verfahren nach einem der Ansprüche 11 bis 13, **dadurch gekennzeichnet, daß** das (die) vorgenannte(n) feste(n) Bindemittel in einer Menge zwischen 5 und 40, vorzugsweise zwischen 10 und 35 Gew.-%, bezogen auf das Gewicht des Fenofibrats verwendet wird (werden).
